# EUROPEAN PATENT APPLICATION

(11) **EP 3 398 608 A1**
(43) Date of publication of application: **07.11.2018**
(21) Application number: 16866696.4
(22) Date of filing: 18.11.2016
(51) Int. Cl.: A61K 38/17, A61K 38/16, A61K 48/00, A23L 33/18

(54) **PHARMACEUTICAL COMPOSITION FOR PREVENTING AND TREATING CANCER OR CANCER METASTASIS, CONTAINING PSTL1 PROTEIN AS ACTIVE INGREDIENT**

(30) Priority: 18.11.2015 KR 20150161900
(71) Applicant: Metacine, Inc., Wonju-si, Gangwon-do 26493 (KR)
(72) Inventor: PARK, Bae Keun, Wonju-si Gangwon-do 26494 (KR)
(74) Representative: Ström & Gulliksson AB
(86) International application number: PCT/KR2016/013358
(87) International publication number: WO 2017/086744

(57) **Abstract**

The present invention relates to a pharmaceutical composition for preventing and treating cancer, cancer metastasis and bone metabolic diseases, which comprises FSTL1 (Follistatin-like 1) protein as an active ingredient. More specifically, FSTL1 inhibits AKT activity and increases activity of tumor suppressor protein PTEN (phosphatase and tensin homolog deleted on chromosome 10), while inhibiting DNA binding of NF-κB (nuclear factor-κB) and blocking the expression of target genes IL-6, GM -CSF, MMP-9, VEGF and ICAM1, and exhibits significant inhibitory effects on breast cancer growth and metastasis in animal models. Therefore, it can be useful for inhibiting cancer metastasis including breast cancer.

## Description

### [Technical Field]

The present invention relates to a composition and method for treating cancer, inhibiting cancer metastasis, or for treating bone metabolic diseases, which comprises FSTL1 (Follistatin-like 1) protein, a vector containing a polynucleotide encoding the protein, cells or its culture fluid comprising thereof as an active ingredient.

### [Background Art]

Cancer remains one of the incurable diseases in spite of devoting efforts of mankind consistently for decades. Cancer is one of the highest threats to human health since it occurs in an unlimited and uncontrolled way of cells are proliferated and immortalized through a series of mutagenic processes. Following to the various biochemical mechanisms related to cancers identified, therapies thereof have being developed. However no fundamental method of cancer treatment has yet been proposed.

Accordingly, needs for the identification of various molecules related to cancers *in vivo* and the development of drugs targeting such molecules continues, and efforts to improve the therapeutic effect of cancers by combining some of these drugs are also being continued. Recently, a number of anticancer drugs such as taxol, rapamycin and 17-allylaminogeldanamycin (17-AAG) have been developed along with the remarkably made in various studies such as cancer cell biology and medicinal chemistry. Anticancer drugs having new action mechanisms such as Gleevec are being developed.

Among various cancers, breast cancer is one of the most studied cancers among all kinds of cancers and it is known to be caused by both environmental factors and genetic factors, but nothing about the cause of breast cancer has yet been clearly identified. However, several studies have presented possible factors of it, and there is no question on that estrogen, a female hormone, plays an important role in the carcinogenesis process. Since breast cells proliferate and differentiate by the stimulation of estrogen, it is thought that the risk of breast cancer in individuals is determined by the time of exposure to estrogen. That is, the longer exposure time to estrogen, the greater incidence rate of breast cancer. In addition, excessive nutrition and fat intake, genetic factors, obesity, long-term contraceptive use that can cause hormonal unbalance, and also a long-term female hormonal therapy are presumed to be the causes of breast cancer.

The patient is brought to death by the cancer due to the cancer metastasis, and particularly, in case of breast cancer, metastasis to bone tissue is often progressive or recurred. In clinical cases, it has been reported that 30 to 40% of early metastasis of breast cancer is metastatic to bone tissue, and more than 90% of patients who is in terminal stage or died had metastasized to bone tissue. There are three main causes of selective bone metastasis: first, the high accessibility of breast cancer cells to bone tissue due to the massive blood flow around the red bone marrow, and second, among others, the expression of adsorption inducing molecules (i.e., a drawing ligand molecules) in bone tissue stromal cells that bind to a homing instinct breast cancer surface molecules (i.e., homing receptor of a breast cancer cell). As the third, calcium (Ca²⁺) and growth factors promoting the proliferation of breast cancer cells are contained in bone tissue. When a single or a small number of breast cancer cells are transferred to bone tissue, osteoclast differentiation is induced and bone destruction (bone resorption) progresses. By those factors released in the process, the proliferation of breast cancer is accelerated (i.e., a vicious cycle), and it ultimately leads to death for most patients.

The progress of bone resorption is made by the action of osteoclasts. The osteoclast differentiation is mediated by the expression of PTHrP (parathyroid hormone-related protein) and GM-CSF (Granulocyte) following to the transcriptional regulation of activated NF-κB (Nuclear Factor-macrophage colony-stimulating factor). Though osteoblasts, the PTHrP increases the RANKL (RANK ligand) which competitively binds to OPG (osteoprotegerin) and RANK (receptor activator of NF-κB) and simultaneously the PTHrP promotes the binding of RANK with RANKL by reducing the expression of OPG (i.e., RANKL antagonist of RANK inhibitor). When the RANKL binds to RANK, the differentiation process of osteoclast precursors is initiated. GM-CSF promotes the formation and differentiation of osteoclast precursors and helps the differentiation of osteoclasts.

NF-κB is a protein that plays a role in cell survival, immune response control, or autoimmune regulation of cancers and inflammations. Like many other transcription factors, NF-κB also functions differently depending on cell types or action timings. In case of breast cancer cells, the NF-κB exists being always active and regulates carcinogenesis, proliferation, and metastasis. IKK (IκB kinase), an enzyme of phosphorylating IκB (Inhibitor-κB), regulates the activation of NF- κB and the activated NF-κB translocates into the nucleus and initiates to fight the target genes.

FSTL1 (Follistatin-like 1) was first identified in the mouse osteoblast cell line MC3T3-E1. It is a gene product induced by TGF-β1 (Transforming growth factor-β1), and is known as TSC-36 (TGF-β1 -stimulated clone 36) which has a homology of 92% or more with human protein. It is reported that FSTL1 is strongly expressed in the heart, placenta, prostate, ovary, and small intestine (cited documents 1 and 2) in human tissue, and FSTL1 in animal embryos is associated with not only mouse kidney and lung development (cited document 3) but also regulation of dorsalization and neural induction of the chicken's mesoderm.

It is also known that expression is reduced in some human cancer cell lines (cited documents 4, 5 and 6), cancer tissues (cited document 7), or cells transformed with tumor genes (cited document 8), however the role of FSTL1 in breast cancer metastasis is currently unknown.

As a result of efforts to develop a novel therapeutic target substance for cancer metastasis, the present inventor has found and confirmed that FSTL1, a target gene of AP-1 (Activator protein 1), inhibits AKT activity and promotes the activity of PTEN (Phosphatase and tensin homolog deleted on chromosome 10), a tumor suppressor protein; inhibits the signaling pathway of NF-κB to block the expression of NF-κB target genes IL-6, GM-CSF, MMP-9, VEGF and ICAM1; and the efficacy of effective inhibition of breast cancer growth and bone metastasis in animal models, and finally the present inventors completed the present invention by confirming that the FSTL1 protein can be effectively used as an effective ingredient of a pharmaceutical composition for inhibiting cancer of metastasis.

### [Citation List]

1. Tanaka, M. et al. Cloning of follistatin-related protein as a novel autoantigen in systemic rheumatic diseases. Int Immunol 10, 1305-14 (1998).
2. Vertegaal, A.C. et al. cDNA micro array identification of a gene differentially expressed in adenovirus type 5- versus type 12-transformed cells. FEBS Lett 487, 151-5 (2000).
3. Adams, D., Larman, B. & Oxburgh, L. Developmental expression of mouse Follistatin-like 1 (Fstl1): Dynamic regulation during organogenesis of the kidney and lung. Gene Expr Patterns 7, 491-500 (2007).
4. Sumitomo, K. et al. Expression of a TGF-beta1 inducible gene, TSC-36, causes growth inhibition in human lung cancer cell lines. Cancer Lett 155, 37-46 (2000).
5. Mashimo, J., Maniwa, R., Sugino, H. & Nose, K. Decrease in the expression of a novel TGF beta1-inducible and ras-recision gene, TSC-36, in human cancer cells. Cancer Lett 113, 213-9 (1997).
6. Hambrock, H.O. et al. Structural characterization of TSC-36/Flik: analysis of two charge isoforms. J Biol Chem 279, 11727-35 (2004).
7. Meehan, K.L., Holland, J.W. & Dawkins, H.J. Proteomic analysis of normal and malignant prostate tissue to identify novel proteins lost in cancer. Prostate 50, 54-63 (2002).
8. Johnston, I.M. et al. Regulation of a multigenic invasion programme by the transcription factor, AP-1: re-expression of a down-regulated gene, TSC-36, inhibits invasion. Oncogene 19, 5348-58 (2000).

### [Summary of Invention]

### [Technical Problem]

It is an object of the present invention to provide a composition and method for the treatment or inhibition of cancer or cancer metastasis and the treatment of bone metabolic diseases, comprising FSTL1 (Follistatin-like 1), a vector comprising a polynucleotide encoding the FSTL1 protein, a cell containing the vector or a culture thereof.

### [Solution to Problem]

In order to accomplish the above object, the present invention provides a pharmaceutical composition comprising FSTL1 (Follistatin-like 1) protein as an active ingredient for preventing and treating cancer or cancer metastasis.

The present invention also provides a pharmaceutical composition comprising a vector comprising a polynucleotide encoding the FSTL1 protein, a cell comprising the vector, or culture fluid thereof as an active ingredient for the prevention and treatment of cancer or cancer metastasis.

In addition, the present invention provides a health food containing FSTL1 protein as an active ingredient for improving cancer or cancer metastasis.

The present invention also relates to a pharmaceutical composition and health food comprising as an active ingredient an FSTL1 protein, a vector comprising a polynucleotide encoding the FSTL1 protein, a cell containing the vector, or culture fluid thereof for the prevention and treatment of bone metabolic diseases (osseous metabolic diseases).

In addition, the present invention provides a screening method of agent for preventing or treating cancer or cancer metastasis comprises:
1) treating the test substance with a cancer cell line as an experimental group;
2) measuring the expression level of FSTL1 (Follistatin-like 1) protein in the treated cell line of step 1); and
3) selecting the test substance whose FSTL1 protein expression in step 2) is increased compared to the control.

### [Advantageous Effects of Invention]

FSTL1, a target gene of AP-1 (Activator protein 1), inhibits AKT activity, increases the activity of PTEN (Phosphatase and tensin homolog deleted on chromosome 10), inhibits NF-κB (nuclear factor- κB), blocks the expression of the target genes IL-6, GM-CSF, MMP-9, VEGF and ICAM1, and exhibits significant inhibition of breast cancer growth and inhibition of bone metastasis in animal models, and thus it can be used for the prevention and treatment of cancer, cancer metastasis, or bone metabolic diseases.

### [Brief Description of Figures]

FIG. 1 shows the expression of FSTL1 (Follistatin-like 1) in the MDA-MB-231 breast cancer cell line (MDA / T) transduced a c-jun/AP-1 mutant (Dominant-Negative Mutant) gene by using retrovirus.
   MDA / V: control, MDA-MB-231 breast cancer cell line transduced with retrovirus:
   protein F: FSTL1 protein.
FIG. 2 is a graph showing inhibition of AKT activity by FSTL1 protein and induction of tumor suppression function of PTEN (phosphatase and tensin homolog deleted on chromosome 10).
FIG. 3 is a graph showing inhibition of DNA binding and transcriptional regulation of NF-κB by FSTL1 protein and blocking of NF-κB target protein expression.
FIG. 4 shows inhibition of adhesion and invasion mechanism of breast cancer cells by FSTL1 protein and blocking effect of tumor angiogenic factor expression *in vitro* models.
FIG. 5 is a graph showing the effect of FSTL1 protein on breast tumor growth, bone metastasis inhibition, and osteoclast-reducing effects in animal models.
FIG. 6 is a graph showing the inhibitory effect of FSTL1 protein on osteoclastogenesis in an *ex vivo* model of mouse bone marrow cells.

### [Description of Embodiments]

Hereinafter, the present invention is described in more detail.

The present invention provides a pharmaceutical composition for preventing and treating cancer comprising FSTL1 (Follistatin-like 1) protein as an active ingredient.

The present invention also provides a pharmaceutical composition for preventing and treating cancer comprising a vector comprising a polynucleotide encoding an FSTL1 protein, a cell containing the vector, or culture thereof as an active ingredient.

The cancer is preferably one or more selected from the group consisting of melanoma, small cell lung cancer, non-small cell lung cancer, glioma, liver cancer, thyroid tumor, gastric cancer, prostate cancer, breast cancer, ovarian cancer, bladder cancer, lung cancer, colorectal cancer, breast cancer, prostate cancer, glioblastoma, endometrial cancer, renal cancer, colon cancer, pancreatic cancer, esophageal cancer, head and neck cancer, mesothelioma, sarcoma, cholangiocarcinoma, small intestine cancer, pediatric malignant cancer and epidermal cancer, more preferably one or more selected from the group specifically consisting of breast cancer, colorectal cancer, pancreatic cancer, prostate cancer, lung cancer, and renal cancer, and more particularly, breast cancer is more preferable a.

The vector may be a linear DNA expressed in human or animal cells, a plasmid vector, a vector containing a viral expression vector or a recombinant retrovirus vector, a recombinant adenovirus vector, and recombinant virus vectors comprising a recombinant adeno-associated virus vector(AAV), a recombinant Herpes simplex virus vector or a recombinant Lentivirus vector, but is not limited thereto.

The cells above stated are preferably selected from the group consisting of hematopoietic stem cells, dendritic cells, autologous tumor cells and established tumor cells, and both the cell itself and the culture medium in which the cells are cultured can be used.

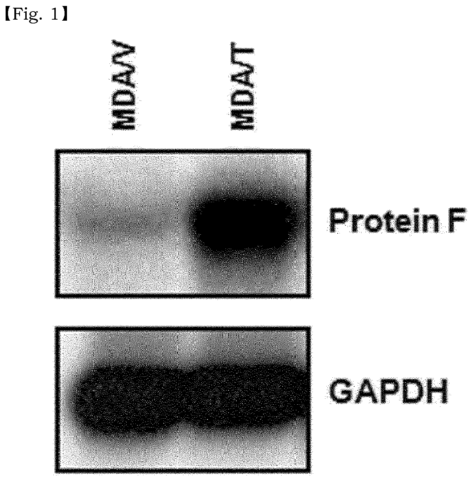

In the specific experimental examples of the present invention, the inventors identified the target protein of the activator protein-1 (AP-1) known to be related with the cancer growth and metastasis, and as a result, the expression of FSTL1 protein was confirmed in the MDA-MB-231 breast cancer cell line transduced with retrovirus expressing the dominant negative mutation of c-jun / AP-1 (see FIG. 1).

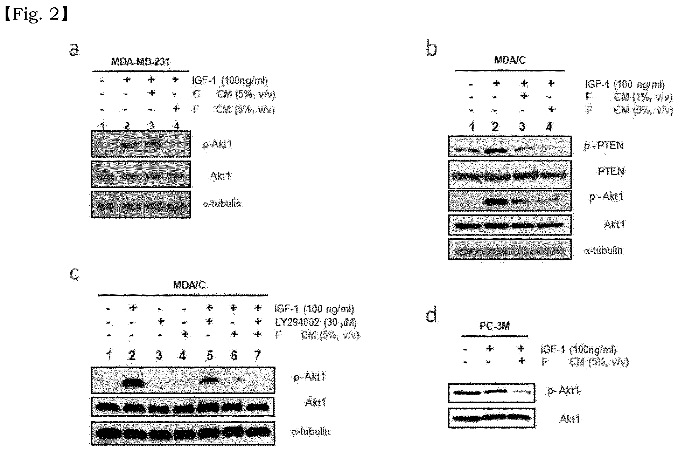

In addition, as a result of identifying the signal pathway of FSTL1 anticancer activity of, confirmed that FSTL1 of the present invention inhibites phosphorylation of AKT1 in breast cancer and metastatic prostate cancer cell lines (see FIGS. 2a and 2d), which indicates that FSTL1 is an excellent inhibitor of AKT activity by inhibiting the activity significantly versus a compound known as PI-3 kinase inhibitor, an upstream signal target protein of AKT (see Fig. 2c). FSTL1 increases the tumor suppression effect by inhibiting the phosphorylation of tumor suppressor PTEN, and the effect increases following to the amount thereof increases (see FIG. 2b).

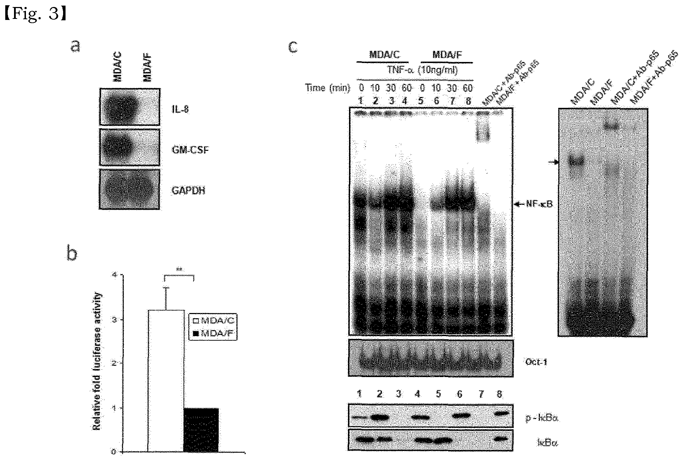

Further, as a result of confirming the regulation effect of inhibiting NF-κB DNA binding in breast cancer cells, confirmed that FSTL1 can inhibit the expression of NF-κB transcriptional gene, thereby it can act as an inhibitor of breast cancer metastasis (see FIG. 3).

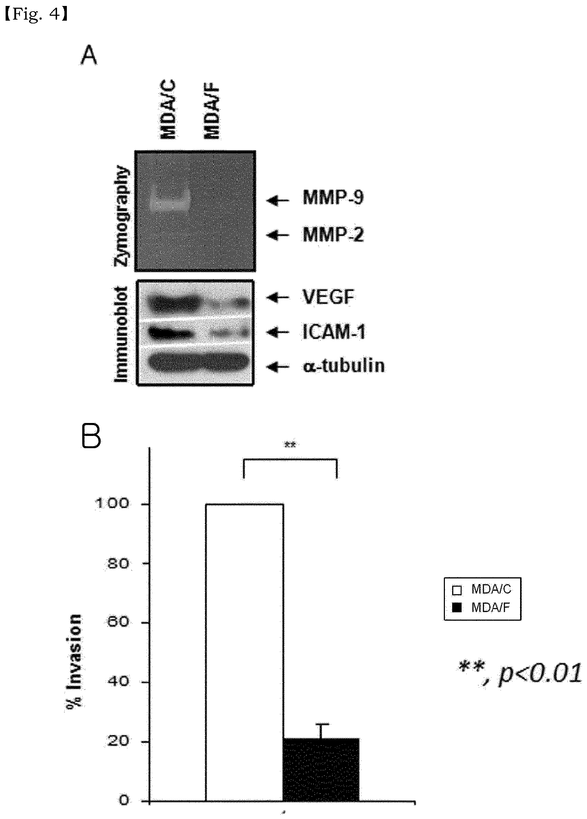

Also, confirmed that FSTL1 significantly inhibites the expression of the adhesion and invasion mechanism mediated molecule of breast cancer cells, and tumor angiogenesis factors those regulated by NF-κB *in vitro* models (see FIG. 4a), and the invasion ability remarkably reduced by about 79% or more (see FIG. 4b).

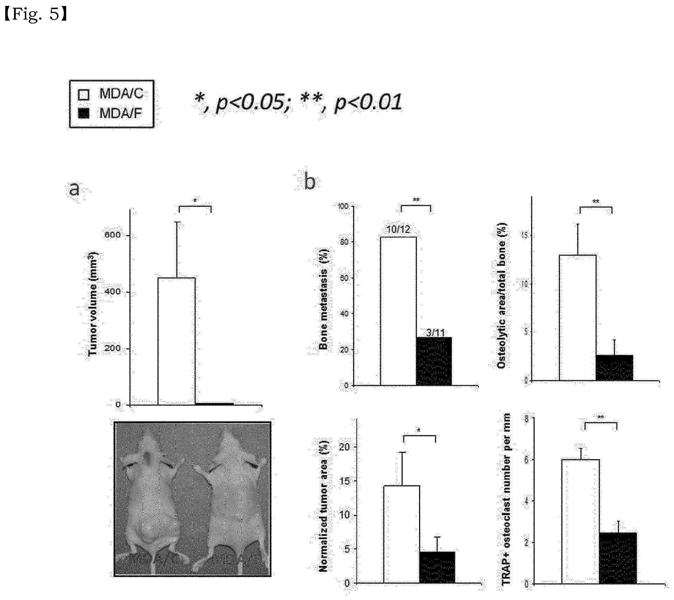

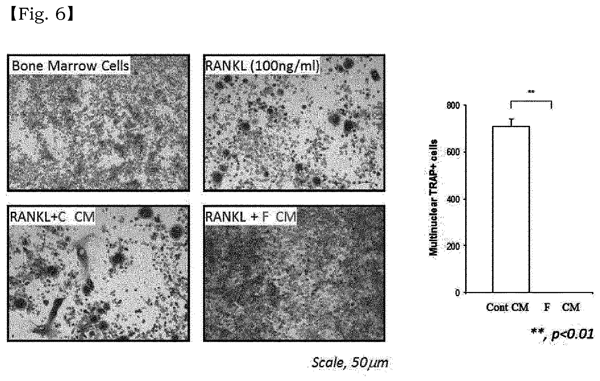

In addition, as a result of confirming the effect of FSTL1 in animal models, confirmed that FSTL1 significantly inhibites tumor growth and breast cancer bone metastasis (see FIG. 5), as well as the quantitative reduction of osteoclasts (see FIG. 5 bottom right) and inhibition of osteoclasts generation in *ex vivo* models (see FIG. 6).

Therefore, FSTL1, AP-1 target gene of the present invention inhibits AKT activity being associated with tumorigenesis and cell viability, increases the function of PTEN, tumor suppressor protein, and inhibits the expression of target protein by interfering the signal pathway of NF-κB, and thus it can be useful in the prevention and treatment of cancer since it represents a significant growth inhibition of breast cancer and bone metastasis inhibitory effect in animal models.

The therapeutically effective amount of the composition of the present invention may vary depending on the variety of factors, such as the method of administration, the site of the subject, the condition of the patient, and the like. Therefore, when used in the human body, the dosage should be determined in consideration of safety and efficacy. It is also possible to estimate the amount to be used in humans from the effective amount determined through animal experiments. These considerations for determining the effective amount are described, for example, in Hardman and Limbird, eds., Goodman and Gilman's The Pharmacological Basis of Therapeutics, 10th ed.(2001), Pergamon Press; and E.W. Martin ed., Remington's Pharmaceutical Sciences, 18th ed.(1990), Mack Publishing Co..

The present composition may also include carriers, diluents, excipients, or a combination of two or more thereof, which are commonly used in biological products. The pharmaceutically acceptable carrier is not particularly limited as long as the composition is suitable for *in vivo* delivery, for example, compounds described in Merck Index, 13th ed., Merck & Co. Inc., a buffered saline solution, sterilized water, Ringer's solution, a buffer solution, a dextrose solution, a maltodextrin solution, glycerol, ethanol, and one or more of these components may be mixed and used, and if necessary, other conventional additives such as an antioxidant, a buffer, bacteriostatic agent, may be added thereto. In addition, a diluent, a dispersing agent, a surfactant, a binder and a lubricant may be additionally added and formulated to a main use formulations such as an aqueous solution, a suspension, an emulsion, etc., a pill, a capsule, a granule or a tablet. Further, it can be suitably formulated according to each disease or ingredient, using the method disclosed in Remington's Pharmaceutical Science (Mack Publishing Company, Easton PA, 18th, 1990), in a suitable manner in the art.

The composition of the present invention may further contain one or more active ingredients showing the same or similar functions. The composition of the present invention contains 0.0001 to 10% by weight, preferably 0.001 to 1% by weight of the protein, based on the total weight of the composition.

The composition of the present invention may be administered parenterally (for example, intravenously, subcutaneously, intraperitoneally or topically) or orally, depending on the intended method, and the dosage varies on the patient's body weight, age, sex, health condition, diet, administration time, administration method, excretion rate, and severity of disease. The daily dose of the composition according to the present invention is 0.0001 to 10 mg / ml, preferably 0.0001 to 5 mg / ml, more preferably once to several times a day.

The vector containing the polynucleotide encoding the FSTL1 protein of the present invention preferably contains 0.05 to 500 mg, more preferably 0.1 to 300 mg, and in case of recombinant virus comprising the polynucleotide encoding the FSTL1, it is preferable to contain 10³-10¹² IU (10-10¹⁰ PFU, more preferably 10⁵-10¹⁰ IU, but is not limited thereto.

In addition, in the case of a cell comprising a polynucleotide encoding the FSTL1 protein of the present invention, it is preferable to contain 10³-10⁸, more preferably 10⁴-10⁷, but is not limited thereto.

In addition, the effective dose of composition of the present invention containing a vector including a polynucleotide encoding the FSTL1 protein or cells as an active ingredient is 0.05 to 12.5 mg per 1 kg of body weight in the case of containing a vector, 10⁷ to 10¹¹ virus particles (10⁵ to 10⁹ IU) per 1 kg of body weightin case of containing recombinant virus, 10³ to 10⁶ cells per 1 kg of body weight in case of cells, preferably 0.1 to 10 mg/kg in case of vector, 10⁸ to 10¹⁰ particles (10⁶ to 10⁸ IU)/kg in case of recombinant virus, 10² to 10⁵ cells/kg in case of cells, and can be administered 2 to 3 times a day. Above stated compositions are not necessarily limited to them, and may vary depending on the condition of the patient and the degree of disease.

The present invention also provides a health food for prevention and improvement of cancers, which comprises an FSTL1 protein as an active ingredient.

FSTL1, AP-1 target gene of the present invention, inhibits AKT activity being associated with tumorigenesis and cell viability, increases the function of PTEN, tumor suppressor protein, and inhibits the expression of target protein by interfering the signal pathway of NF-κB, and thus it can be effectively used as an effective ingredient of health food for cancer prevention and improvement since it represents significant efficacy on growth inhibition of breast cancer and bone metastasis inhibitory activities in animal models.

The present invention also provides a pharmaceutical composition for inhibiting cancer metastasis comprising FSTL1 protein as an active ingredient.

The present invention also provides a pharmaceutical composition comprising a vector including a polynucleotide encoding an FSTL1 protein, a cell comprising the vector, or culture fluid thereof as an active ingredient for inhibiting cancer metastasis.

It also provides health food comprising a FSTL1 protein, a vector comprising a polynucleotide encoding the FSTL1 protein, a cell containing the vector, or culture fluid thereof as an active ingredient for improving cancer metastasis.

The cancer is preferably one or more selected from the group consisting of melanoma, small cell lung cancer, non-small cell lung cancer, glioma, liver cancer, thyroid tumor, gastric cancer, prostate cancer, breast cancer, ovarian cancer, bladder cancer, lung cancer, colorectal cancer, breast cancer, prostate cancer, glioblastoma, endometrial cancer, renal cancer, colon cancer, pancreatic cancer, esophageal cancer, head and neck cancer, mesothelioma, sarcoma, cholangiocarcinoma, small intestine cancer, pediatric malignant cancer and epidermal cancer, more preferably one or more selected from the group consisting of breast cancer, colorectal cancer, pancreatic cancer, prostate cancer, lung cancer, and renal cancer, and more particularly, it is more preferably a breast cancer, but is not limited thereto.

In the specific experimental examples of the present invention, confirmed that FSTL1 of the present invention significantly inhibits the expression of MMP-9, VEGF and ICAM-1, molecules being related with cancer metastasis (see Table 1 and FIG. 4).

Therefore, according to the present invention, FSTL1, a target gene of AP-1 (Activator protein 1), inhibits AKT activitities being associated with tumorigenesis, cell proliferation and cell viability; increases the activity of PTEN, tumor suppressor agent;inhibit NF-κB signal pathway; blocks the expression of the target genes IL-6, GM-CSF, MMP-9, VEGF and ICAM1, target gene of NF-κB, and exhibits significant inhibition of breast cancer growth and inhibition of bone metastasis in animal models, and thus it can be used as an active ingredient of composition for inhibiting cancer metastasis.

The present invention also provides a pharmaceutical composition comprising as an active ingredient a FSTL1 protein, a vector comprising a polynucleotide encoding the FSTL1 protein, a cell containing the vector, or culture fluid thereof for the prevention and treatment of bone metabolic diseases.

The present invention also provides health food comprising a FSTL1 protein, a vector comprising a polynucleotide encoding the FSTL1 protein, a cell containing the vector, or culture fluid thereof as an active ingredient for the prevention and improvement of bone metabolic diseases.

The bone metabolic diseases include preferably any one selected from the group consisting of bone metastatic cancer, solid cancer bone metastasis, musculoskeletal complications due to solid cancer metastasis, hypercalcemia due to malignant tumors, multiple myeloma, primary bone tumors, osteoporosis, rheumatoid arthritis, degenerative arthritis, periodontal disease, inflammatory alveolar bone disease, inflammatory bone resorption disease and Paget's disease, most preferably a breast cancer bone metastasis.

FSTL1 according to the present invention, a target gene of AP-1, inhibits AKT activity being associated with tumorigenesis, cell proliferation and cell viability; increases the activity of PTEN,a tumor suppressor agent; inhibits NF-κB signal pathway; blocks the expression of the target genes IL-6, GM-CSF, MMP-9, VEGF and ICAM1, target gene of NF-κB, and exhibits significant inhibition of breast cancer growth and inhibition of bone metastasis in animal models, and thus it can be used as an active ingredient of composition for preventing and treating bone metabolic diseases including breast cancer bone metastasis.

In addition, the present invention provides a screening method of agent for preventing or treating cancer or cancer metastasis comprises:
1) treating the test substance with a cancer cell line as an experimental group;
2) measuring the expression level of FSTL1 (Follistatin-like 1) protein in the treated cell line of step 1); and
3) selecting the test substance whose FSTL1 protein expression in step 2) is increased compared to the control.

Preferably, the cancer is one or more selected from the group consisting of breast cancer, colorectal cancer, pancreatic cancer, prostate cancer, and renal cancer, and the cancer metastasis is preferably breast cancer bone metastasis, but is not limited thereto.

Preferably, the cancer cell of step 1) expresses AP-1 (Activator protein-1), and the FSTL1 protein of step 2) inhibits AKT activity, activates PTEN (phosphatase and tensin homolog deleted on chromosome 10), and interferes the signal pathway of NF-kB.

The expression level of the FSTL1 protein in the step 2) can be measured by reverse transcription-polymerase chain reaction (RT-PCR), enzyme immunoassay (ELISA), immunohistochemistry, Western blotting and flow cytometry (FACS), but is not limited thereto.

FSTL1 according to the present invention, a target gene of AP-1, inhibits AKT activity being associated with tumorigenesis, cell proliferation and cell viability;, increases the activity of PTEN a tumor suppressor agent, inhibits NF-κB signal pathway; blocks the expression of the target genes IL-6, GM-CSF, MMP-9, VEGF and ICAM1, target gene of NF-κB, and exhibits significant inhibition of breast cancer growth and inhibition of bone metastasis in animal models. Therefore, a substance that increases the expression of FSTL1 in cancer cells can be used to a therapeutic agent or therapeutic supplements for cancer or cancer metastasis.

### [Examples]

Hereinafter, the present invention is described in detail with reference to Examples and Experimental Examples. However, the following Examples and Experimental Examples are merely illustrative of the present invention, and the present invention is not limited to the following Examples and Experimental Examples.

### <Example 1 > Culture of breast cancer cell lines and prostate cancer cell lines

Human breast cancer cells (MDA-MB-231 cells) and prostate cancer cells (PC-3M cells) used in the present invention were purchased from the American Type Culture Collection (ATCC, Rockville, MD, USA). Cells were cultured in a medium including Dulbecco's modified Eagle's medium / Nutrient Mixture Ham's F12 (DMEM / F12, Gibco / BRL, Gaithersburg, MD, USA), 10% FBS, 100 units / ml penicillin, 100 µg / mL Streptomycin (Gibco / BRL). When cell number (i.e., cell density) reaches 80-90% of a 10-cm culture dish, it is washed twice with phosphate buffered saline (PBS) and treated with Trypsin-EDTA (Gibco / BRL). Alternatively, samples were prepared for *in vivo* or *in vitro* experiments, and cell culture medium was changed every 2 to 3 days.

### <Example 2> Identification of Target Protein of Transcription Factor AP-1 (Activator protein-1)

In order to analyze the pattern of intracellular gene expression by the transcription factors AP-1 and FSTL1 known to be related to the growth and metastasis of cancers, the dominant-negative mutant gene of c-jun / AP-1 or the FSTL1 clone was analyzed in a Gene pool showing increased expression or decreased expression respectively in each MDA-MB-231 breast cancer cell line transduced with retrovirus by Northern blotting and cDNA microarray analysis (i.e., Human genome-U133 plus 2.0 genechip , Affymetrix, Inc.).

As a result, as shown in Fig. 1, confirmed that the FSTL1 gene was up-expressed by the introduction of the dominant negative mutation of c-jun / AP-1 and the FSTL1 gene and protein expression was inhibited in the control breast cancer cell line (Fig. 1).

Therefore, confirmed that FSTL1 protein is correlated with tumor suppression.

### <Experimental Example 1> Identification of FSTL1 antitumor activity signal pathway

The FSTL1 condition medium (5%, v/v) and 100 ng/ml of IGF-1(insulin-like factor-1) were treated to the breast cancer cell lines (MDA-MB-231 cell), prostate cancer cell lines (PC-3M) cultured in Example 1, or a MDA-MB0231 breast cancer cell lines transfected with retrovirus as a control, and then assessed Akt 1 and PTEN(phosphatase and tensin homolog deleted on chromosome 10) activities by using Western blotting. On the other hand, PI-3K (phosphatidylinositol-3 kinase) inhibitor or Akt inhibitor LY294002 was used as a control.

As a result, as shown in FIG. 2, the FSTL1 condition medium of the present invention inhibited the phosphorylation of Aktl (FIGS. 2a and 2d) and inhibited phosphorylation of the cancer inhibitor PTEN, an upstream signal target of PI-3K, and increased tumor suppression effect, and specifically the FSTL1 condition medium of the present invention significantly inhibited the phosphorylation of Aktl than the compound known as the conventional PI-3K inhibitor or Akt inhibitor (FIG. 2b).

### <Experimental Example 2> Identification of NF-κB Signal Pathway Regulation of FSTL1

In order to confirm the NF-κB signal pathway of FSTL1, genes down-regulated by FSTL1 were analyzed using Human Genome-U133 Plus 2.0 Genechip and Affymetrix.

As a result, as shown in Table 1, it was confirmed that the gene down-regulated by FSTL1 is a gene having functions such as angiogenesis, cell adhesion / migration, cell growth, inflammation and tumor formation regulation and the like (Table 1).

**[Table 1]**

| Function | Gene ID | Gene Symbol, Description | Fold Change |
|---|---|---|---|
| Angiogenesis | AF043337.1 | IL8, Interleukin8 | 13.76 |
| | NM_000600.1 | IL6, Interleukin 6 (interferon, beta 2) | 4.21 |
| | S69738.1 | CCL2, Monocyte chemotactic protein 1 | 18.35 |
| Cell Adhesion | NM_002207.1 | ITGA9, Integrin, alpha 9 | 15.00 |
| Cell Growth | NM_001511.1 | CXCL1, GRO1 oncogene | 10.78 |
| Development | NM_006576.1 | AVIL, Advillin | 2.47 |
| Inflammation | NM_000064.1 | C3, Complement component 3 | 34.58 |
| | M57731.1 | CXCL2, GRO-beta | 6.49 |
| | NM_002090.1 | CXCL3, GRO3 oncogene | 7.77 |
| Stimulation | M11734.1 | CSF2, GM-CSF | 63.11 |
| | NM_000759.1 | CSF3,G-CSF | 17.14 |
| Tumorigenesis | NM_002387.1 | MCC, mutated in colorectal cancers | 3.08 |
| | Al829726 | CCND1, Cyclin D1 | 4.09 |

| | | | |
|---|---|---|---|
| Human Genome-U133 Plus 2.0 Gene Chip, Affymetrix, Inc. | | | |

At the mRNA level, FSTL1 also blocked the expression of IL-8 (interleukin-8) cytokines regulated by NF-κB, and a granulocyte macrophage colony stimulating factor (GM-CSF)(Fig. 3a).

In addition, it was confirmed that FSTL1 quantitatively inhibits the binding of NF-kB with cytokine promoter through Luciferase reporter analysis (Fig. 3b).

Furthermore, it was confirmed that FSTL1 interferes the transcriptional activity of NF-κB through an electromobility gel shift assay (EMSA) (FIG. 3c). However, it was confirmed that FSTL1 does not follow the TNF-α dependent signal pathway. Following to the FSTL1 treatment, IκBα was not only degraded, but also damaged the phosphorylation of Akt and IκBα.

Therefore, it was confirmed that FSTL1 can regulate the induction of Akt-mediated NF-κB signaling and play a role as an inhibitor of breast cancer metastasis.

### <Experimental Example 3> Confirmation of inhibitory effect of FSTL1 on NF-κB signal pathway

Expression of various FSTL1-mediated molecules (MMP-9, VEGF, ICAM-1) was analyzed. These molecules are regulated by NF-κB and are involved in metastasis-related invasion, angiogenesis and cell adhesion. In particular, MMP-9 has been implicated in tumor angiogenesis and metastasis as well as activation of growth factor required for cancer which stimulates breast cancer activity. In addition, MMP-9 is expressed by osteoclasts and is required for osteoclast migration. Therefore, MMP-9 plays an important role in the osteoclast microenvironment being associated with bone resorption.

The expression of MMP-9, VEGF, and ICAM-1 was analyzed by immunoblot and zymography using the breast cancer cell lines expressing FSTL1 and control breast cancer cell lines prepared in Example 2 above.

As a result, as shown in Fig. 4, it was confirmed that FSTL1 of the present invention significantly inhibited all of the factors *in vitro* (Fig. 4a) and remarkably decreased FSTL1-mediated invasion ability by about 79% or more (Fig. 4b).

### <Experimental Example 4> Confirmation of effect of FSTL1 in animal model

### <4-1> Preparation of experimental animals

Nude mice were BALB /c/nu (Korea Research Institute of Bioscience and Biotechnology, Korea) as a basic species, and all 20 females were 6 ∼ 8 weeks old and weighing about 20 g. Sterilized water and feed were supplied in sterilized cage, and a clean environment was maintained by placing the lamina flow in a continuously held clean bench, and all procedures were also carried out in clean bench. For the day and night biological cycle of nude mice, the interior lights were illuminated for 12 hours and blinked for 12 hours.

### <4-2> Identification of tumor growth inhibitory effect

The breast cancer cell line expressing FSTL1 prepared in Example 2 above was inoculated subcutaneously into a nude mouse using a 1 cc syringe and a 25 gauge needle to prepare a nude mouse prepared in Experimental Example <4-1> , and then nude mice transplanted with breast cancer cell lines and tumor volume were measured. Tumor volume was measured by caliper length and width once every 3 days, and the tumor volume was calculated by the formula of Gutman et al.

As a result, as shown in FIG. 5a, it was confirmed that the mice transplanted with the breast cancer cell line expressing FSTL1 significantly inhibited tumor growth (FIG. 5a).

### <4-3> Confirmation of osteolytic bone metastasis inhibitory effect

As shown in FIG. 5b, it was confirmed that FSTL1 of the present invention suppressed bone metastasis by about 67% or more (FIG. 5b).

### <Experimental Example 5> Confirmation of inhibitory effect of FSTL1 on osteoclastogenesis

### <5-1> Culture of mouse bone marrow cells

ICR mouse (6-9 weeks, male) was cervical dislocated and disinfected with 70% ethanol. The skin of the tibia part was dissected and the attachment muscles were removed. The tibia was cut and the patella was dislocated to remove the tibia. The bone was cut a little bit and a 25G needle was inserted into one end of the bone, and α-MEM was poured to collect the bone marrow cells in the test tube. After centrifugation, the cells were suspended in α-MEM and 2 times of Gey's solution were added to remove the red blood cells. After centrifugation, the cells were resuspended in α-MEM containing 10% FBS.

### <5-2> Confirmation of inhibitory effect on osteoclastogenesis

The bone marrow cells cultured in the above <5-1> were inoculated in a 96-well plate at 1 × 10³ cells. Then, after FSTL1 CM of the present invention was pretreated, RANKL was treated, and after 7 days of incubation in a 5% CO₂ incubator, stained with TRAP (tartrate resistance acid phosphatase), and the number of osteoclast differentiated cells was counted to confirm the inhibition of osteoclast differentiation by FSTL1 CM.

As a result, as shown in Fig. 6, it was confirmed that the FSTL1 CM of the present invention significantly inhibited osteoclast formation (Fig. 6).

## Claims

1. A pharmaceutical composition comprising FSTL1 (Follistatin-like 1) protein as an active ingredient for preventing and treating cancer or cancer metastasis.

2. A pharmaceutical composition comprising a vector including a polynucleotide encoding an FSTL1 protein, a cell containing the vector, or culture fluid thereof as an active ingredient for preventing or treating cancer or cancer metastasis.

3. The pharmaceutical composition according to claim 1 or 2, wherein the cancer is at least one selected from the group consisting of breast cancer, colorectal cancer, pancreatic cancer, prostate cancer, and renal cancer.

4. The pharmaceutical composition according to claim 2, wherein the vector said is linear DNA, plasmid DNA or recombinant viral vector.

5. The pharmaceutical composition according to claim 4, wherein the recombinant virus is any one selected from the group consisting of retrovirus, adenovirus, adeno-associated virus, Herpes simplex virus and Lentivirus..

6. The pharmaceutical composition according to claim 2, wherein the cell is any one selected from the group consisting of hematopoietic stem cells, dendritic cells, autologous tumor cells, and established tumor cells.

7. Health food comprising FSTL1 protein as an active ingredient for preventing or ameliorating cancer or cancer metastasis.

8. A pharmaceutical composition comprising FSTL1 (Follistatin-like 1) protein as an active ingredient for the prevention and treatment of bone metabolic diseases.

9. A pharmaceutical composition comprising a vector comprising a polynucleotide encoding an FSTL1 protein, a cell containing the vector or culture fluid thereof as an active ingredient for the prevention and treatment of bone metabolic diseases.

10. The pharmaceutical composition according to claim 8 or 9, wherein the bone metabolic disease is at least one selected from the group consisting of bone metastatic cancer, solid cancer bone metastasis, musculoskeletal complications due to solid tumor metastasis, hypercalcemia due to malignant tumors, multiple myeloma, primary bone tumors, osteoporosis, rheumatoid arthritis, degenerative arthritis, periodontal disease, inflammatory alveolar bone disease, inflammatory bone resorption disease and Paget's disease.

11. Health food containing FSTL1 protein as an active ingredient for preventing and improving bone metabolic diseases.

12. A screening method of agent for preventing or treating cancer or cancer metastasis comprises:
1) treating the test substance with a cancer cell line as an experimental group;
2) measuring the expression level of FSTL1 (Follistatin-like 1) protein in the treated cell line of step 1); and
3) selecting the test substance whose FSTL1 protein expression in step 2) is increased compared to the control.

13. The method according to claim 12, wherein the cancer is at least one selected from the group consisting of breast cancer, colorectal cancer, pancreatic cancer, prostate cancer, and renal cancer.

14. The method of claim 12, wherein the cancer metastasis is breast cancer bone metastasis.

15. The method according to claim 12, wherein the cancer cells of step 1) express AP-1 (Activator protein-1).

16. The method according to claim 12, wherein the FSTL1 protein of step 2) inhibits AKT activity and increases the activity of PTEN (phosphatase and tensin homolog deleted on chromosome 10).

17. The method according to claim 12, wherein the FSTL1 protein of step 2) blocks the signal pathway of NF-kB.

18. The method according to claim 12, wherein the degree of expression of the FSTL1 protein in step 2) is determined by any one selected from the group consisting of reverse transcription-polymerase chain reaction (RT-PCR), enzyme immunoassay (ELISA), immunohistochemistry, Western blotting and flow cytometry (FACS).

19. A method for treating cancer, comprising administering a pharmaceutically acceptable amount of a FSTL1 protein, a vector comprising a polynucleotide encoding said protein, or a cell comprising said vector or culture fluid thereof to a subject having the cancer.

20. A method for inhibiting cancer metastasis comprising administering a pharmaceutically acceptable amount of a FSTL1 protein, a vector comprising a polynucleotide encoding said protein, a cell comprising said vector, or culture fluid thereof to a subject having the cancer.

21. A method for preventing cancer, which comprises administering a pharmaceutically acceptable amount of a FSTL1 protein, a vector comprising a polynucleotide encoding said protein, a cell comprising said vector, or culture fluid thereof to a subject.

22. A method for preventing cancer metastasis comprising administering a pharmaceutically acceptable amount of an FSTL1 protein, a vector comprising a polynucleotide encoding said protein, or a cell comprising said vector or a culture thereof to a subject.

23. A method for treating a bone metabolic disease which comprises administering a pharmaceutically acceptable amount of a FSTL1 protein, a vector comprising a polynucleotide encoding said protein, a cell comprising said vector, or culture fluid thereof to a subject suffering from a bone metabolic disease.

24. A method for preventing bone metabolic diseases comprising administering a pharmaceutically acceptable amount of a FSTL1 protein, a vector comprising a polynucleotide encoding said protein, a cell comprising said vector, or culture fluid thereof to a subject.

25. Use of the FSTL1 protein for use as a pharmaceutical composition for the prevention or treatment of cancer, cancer metastasis, or bone metabolic diseases.

26. Use of the FSTL1 protein for use as health food for the prevention or amelioration of cancer, cancer metastasis, or bone metabolic disease.
